Europäisches Patentamt

European Patent Office

Office européen des brevets

(19)

(11) **EP 1 201 224 A1**

## (12) DEMANDE DE BREVET EUROPEEN

(43) Date de publication:
 **02.05.2002 Bulletin 2002/18**

(51) Int Cl.⁷: **A61K 7/06**, A61K 7/48

(21) Numéro de dépôt: **01402660.3**

(22) Date de dépôt: **15.10.2001**

(84) Etats contractants désignés:
 **AT BE CH CY DE DK ES FI FR GB GR IE IT LI LU MC NL PT SE TR**
 Etats d'extension désignés:
 **AL LT LV MK RO SI**

(30) Priorité: **30.10.2000 FR 0013937**

(71) Demandeur: **L'OREAL**
 **75008 Paris (FR)**

(72) Inventeurs:
 • **Dupuis, Christine**
 **75018 Paris (FR)**

 • **Samain, Henri**
 **91570 Bievres (FR)**
 • **Lion, Bertrand**
 **95270 Luzarches (FR)**

(74) Mandataire: **Bourdeau, Françoise**
 **L'OREAL,**
 **Département Propriété Industrielle,**
 **6, rue Bertrand Sincholle**
 **92585 Clichy Cedex (FR)**

### (54) Composition cosmétique procurant de bonnes propriétés de tenue et comprenant un copolymère à motif acide

(57) L'invention a pour objet une composition cosmétique comprenant, dans un milieu cosmétiquement acceptable, au moins un polymère filmogène (A) choisi de telle sorte que le matériau obtenu par séchage de ce polymère en milieu aqueux ou alcoolique, à température ambiante et à un taux d'humidité relative de 50 %, présente un profil mécanique défini par au moins:

(a) un taux d'allongement à la rupture ($\varepsilon_r$) supérieur ou égal à 160 %;
(b) une recouvrance instantanée ($R_i$) comprise entre 25 et 70 % après un allongement de 150 %,
(c) une recouvrance ($R_{300}$) à 300 secondes comprise entre 30 et 100 % après un allongement de 150 % ;

le polymère filmogène (A) contenant au moins un monomère à motif acide insaturé, dont l'indice acide est supérieur à 82.

**EP 1 201 224 A1**

**Description**

L'invention a pour objet une composition cosmétique comprenant, dans un milieu cosmétiquement acceptable, au moins un polymère (A) aux caractéristiques particulières. Elle vise également un procédé de mise en forme ou de maintien des cheveux à l'aide de cette composition, ainsi que son utilisation pour la formulation de produits de coiffage tels que les laques, les sprays ou les mousses, en vue d'obtenir le maintien ou la mise en forme de la coiffure.

Parmi les produits capillaires pour la fixation des cheveux les plus répandus sur le marché de la cosmétique, on peut citer les compositions à pulvériser en aérosol ou en flacon pompe tels que les laques, les sprays ou les mousses, essentiellement constituées d'une solution le plus souvent alcoolique ou hydroalcoolique et d'un polymère filmogène soluble dans l'eau ou dans l'alcool, en mélange avec divers adjuvants cosmétiques.

Toutefois, ces formulations capillaires telles que les mousses, gels et surtout les sprays et les laques aérosols destinées à maintenir la forme de la coiffure ne permettent encore pas à la coiffure de résister de manière totalement satisfaisante aux différents mouvements naturels de la vie comme la marche, les mouvements de tête ou les coups de vent.

Les polymères utilisés pour la formulation de ces produits capillaires sont des polymères filmogènes anioniques, amphotères ou non ioniques, qui conduisent à la formation de films possédant un caractère plus ou moins dur et cassant.

Lorsque le polymère est trop cassant, le pourcentage d'allongement à la rupture mesuré sur le film est faible, c'est-à-dire en général inférieur à 2 % et la tenue de la coiffure n'est pas assurée dans le temps.

Pour remédier à ce problème, on a déjà associé ces polymères à des plastifiants et obtenu des revêtements plus souples et non friables. Toutefois, ces films sont déformables et plastiques, c'est-à-dire qu'après déformation, ils ne récupèrent que très peu de leur forme initiale. Si la tenue de la coiffure est améliorée, elle n'est pas encore totalement satisfaisante puisque la forme de la coiffure évolue dans le temps.

Des résultats plus satisfaisants en terme de tenue ont été obtenus avec des compositions comprenant une association de polymères filmogènes, tels que par exemple un polymère polyvinylcaprolactame et un polymère acrylique. Toutefois, ces compositions ne donnent encore pas entièrement satisfaction, dans la mesure où les cheveux perdent certaines de leurs propriétés cosmétiques naturelles. En outre, il serait souhaitable d'augmenter encore le pouvoir fixant.

Par ailleurs, les polymères utilisés jusqu'à présent dans les compositions de coiffage peuvent présenter l'inconvénient de mal s'éliminer aux shampooing.

On recherche donc des compositions cosmétiques pour le maintien et/ou la fixation de la coiffure qui procurent à la chevelure, outre une fixation durable, de bonnes propriétés cosmétiques, notamment un bon démêlage, de la douceur et un aspect agréable et naturel, et qui soient plus performants que ceux de l'art antérieur sur le critère de l'élimination au shampooing.

De manière surprenante et inattendue, la Demanderesse a découvert qu'il était possible de remédier aux problèmes techniques évoqués ci-dessus, en utilisant des polymères particuliers.

L'invention a pour objet une composition cosmétique comprenant, dans un milieu cosmétiquement acceptable, au moins un polymère filmogène (A) choisi de telle sorte que le matériau obtenu par séchage de ce polymère en milieu aqueux ou alcoolique, à température ambiante et à un taux d'humidité relative de 50 %, présente un profil mécanique défini par au moins:

   (a) un taux d'allongement à la rupture ($\varepsilon_r$) supérieur ou égal à 160 %;
   (b) une recouvrance instantanée ($R_i$) comprise entre 25 et 70 % après un allongement de 150 %,
   (c) une recouvrance ($R_{300}$) à 300 secondes comprise entre 30 et 100 % après un allongement de 150 % ;

   le polymère filmogène (A) contenant au moins un monomère à motif acide insaturé, dont l'indice acide est supérieur à 82.

Un autre objet de la présente invention concerne un procédé de mise en forme ou de maintien de la coiffure comprenant la mise en oeuvre de cette composition.

Encore un autre objet de la présente invention concerne l'utilisation de cette composition pour la fabrication de compositions cosmétiques, notamment des compositions capillaires destinées au maintien ou à une mise en forme de la coiffure.

Encore un autre objet de la présente invention concerne un procédé de fabrication de polymères filmogènes et les compositions cosmétiques les contenant.

Au sens de la présente invention, on entend par film obtenu par séchage à température ambiante ($22 \pm 2°C$) et à un taux d'humidité relative de $50 \% \pm 5\%$, le film obtenu dans ces conditions à partir d'un mélange à 6 % de matière active (m.a.) de polymère A avec de l'éthanol ou de l'eau, la quantité de mélange étant adaptée pour obtenir dans une matrice en téflon, un film d'épaisseur de $500 \pm 50$ µm. Le séchage est poursuivi jusqu'à ce que le poids du film n'évolue plus, ce qui représente environ 12 jours. Les polymères A solubles ou partiellement solubles dans l'éthanol sont testés dans l'éthanol. Les autres polymères sont testés dans l'eau sous forme soluble ou dispersée.

Au sens de la présente invention, le taux d'élongation à la rupture et le taux de recouvrance sont évalués aux moyens des essais décrits ci-après.

Pour effectuer les essais de traction, le film est découpé en éprouvettes de forme rectangulaire, de longueur 80 mm et de largeur 15 mm.

Les essais sont réalisés sur un appareil commercialisé sous l'appellation Lloyd, référence LR5K, ou commercialisé sous l'appellation Zwick dans les mêmes conditions de températures et d'humidité que pour le séchage, c'est-à-dire une température de $22 \pm 2$ °C et un taux d'humidité relative de $50 \pm 5$ %.

Les éprouvettes sont étirées à la vitesse de 20mm/mn et la distance entre les mors est de $50 \pm 1$ mm.

Pour déterminer la recouvrance instantanée ($R_i$), on procède comme suit:

- on étire l'éprouvette de 150 % ($\varepsilon_{max}$) c'est-à-dire 1,5 fois sa longueur initiale ($l_0$)
- on relâche la contrainte en imposant une vitesse de retour égale à la vitesse de traction, soit 20mm/mn et on mesure l'allongement de l'éprouvette en pourcentage , après retour à charge nulle ($\varepsilon_i$).

La recouvrance instantanée en % ($R_i$) est donnée par la formule ci-après:

$$R_i = ((\varepsilon_{max} - \varepsilon_i)/ \varepsilon_{max}) \times 100$$

Pour déterminer la recouvrance à 300 secondes, on maintient à contrainte nulle pendant 300 secondes supplémentaires, l'éprouvette ayant subi les opérations précédentes, et on mesure son taux d'allongement en pourcentage ($\varepsilon_{300}$).

La recouvrance à 300 secondes en % ($R_{300}$) est donnée par la formule ci-après:

$$R_{300} = ((\varepsilon_{max} - \varepsilon_{300})/ \varepsilon_{max}) \times 100$$

Dans les compositions conformes à l'invention, le ou les polymères filmogènes (A) sont, de préférence, présents à des concentrations comprises entre 0,05 et 20 % en poids, plus préférentiellement comprises entre 0,1 et 15 % en poids, et plus préférentiellement entre 0,25 et 10 % en poids par rapport au poids total de la composition.

De manière avantageuse, le polymère filmogène (A) est choisi parmi ceux possédant, parmi leurs monomères, au moins un acide insaturé carboxylique ou sulfonique.

Le polymère filmogène (A) comprend, de préférencé, en tant que motif acide insaturé, un motif acide carboxylique, et en particulier l'acide acrylique ou méthacrylique.

De préférence, l'indice acide est compris entre 82 et 235, et plus préférentiellement entre 90 et 190, et encore plus préférentiellement entre 100 et 130.

Avantageusement, le taux d'allongement à la rupture ($\varepsilon_r$) est compris entre 160 et 10 000 %.

De préférence, le (ou les) polymère filmogènes (A) sont solubles en milieu aqueux ou hydroalcoolique. Le (ou les) polymère(s) filmogène (A) peuvent être totalement ou partiellement neutralisés.

Les polymères de l'invention peuvent être fabriqués, de manière non limitative, en effectuant les opérations suivantes, dans un milieu solvant alcoolique ou hydroalcoolique, en présence d'un initiateur de radicaux libres, à une température comprise entre 60 °C et 90 °C

- on polymérise, d'abord, des monomères (i) à motif(s) hydrophile(s), afin d'obtenir un polymère riche en motif(s) hydrophile(s) ;
- on polymérise, ensuite, le polymère riche en motif(s) hydrophile(s) ainsi obtenus avec un mélange de monomères durs (ii) et de monomères mous (iii), afin d'obtenir le polymère filmogène ;

les monomères (i), (ii) et (iii) étant mis en oeuvre dans les proportions suivantes, définies en poids par rapport au poids total des monomères dans le polymère filmogène :

- 10 à 50 % de monomères (i) à motif(s) hydrophile(s) ;
- 20 à 50 % de monomères durs (ii), et
- 30 à 70 % de monomères mous (iii).

Au sens de la présente invention, on entend par *« monomère à motif(s) hydrophile(s) »*, des monomères à motif(s) acide(s) mono- et/ou dicarboxylique, contenant 3 à 8 atomes de carbone, tels que notamment l'acide acrylique, l'acide méthacrylique, l'acide itaconique, et préférentiellement l'acide acrylique.

Par « *monomères durs »,* on entend les méthacrylates d'alkyles linéaires en C1 à C3, cycliques en C6 à C8 et ramifiés, choisis parmi lesdits méthacrylates formant, par polymérisation telle que décrite dans l'ouvrage « Chimie Macromoléculaire - G. Champetier - Edition Hermann », un homopolymère dont la température de transition vitreuse est supérieure à 50°C. On préfère, tout particulièrement, en tant que monomère dur, le méthacrylate de méthyle.

Par « *monomères mous »,* on entend les acrylates linéaires en C1à C 12, cycliques en C6 à C7 et ramifiés, choisis parmi lesdits acrylates formant, par polymérisation telle que décrite dans l'ouvrage « Chimie Macromoléculaire - G.Champetier - Edition Hermann », un homopolymère dont la température de transition vitreuse est inférieure à 25°C. On préfère, tout particulièrement, en tant que monomère mous, les acrylates de méthyle et d'isobutyle.

Avantageusement, on met en oeuvre les monomères (i), (ii) et (iii) dans les proportions suivantes, définies en poids par rapport au poids total des monomères :

- 15 à 30 % de monomères (i) à motif(s) hydrophile(s) ;
- 25 à 40 % de monomères durs (ii), et
- 35 à 55 % de monomères mous (iii).

Le polymère filmogène obtenu par polymérisation des monomères (i), (ii), (iii) est caractérisé par sa masse moléculaire devant, de préférence, être comprise entre 40000 et 150000, et plus préférentiellement entre 80000 et 110000.

En tant qu'initiateur de radicaux libres, on utilise, de préférence, le 2,5-bis (2-éthylhexanoylperoxy)-2,5 diméthylhexane.

La proportion de l'initiateur de radicaux libres est, de préférence, comprise entre 0,6 et 2 % en poids par rapport au poids des monomères et, préférentiellement, voisine de 1,1 %.

Le solvant utilisé, pour mettre en oeuvre le procédé selon l'invention, est préférentiellement l'éthanol.

La proportion de solvant est, de préférence, comprise entre --40-- et -60-- % par rapport au poids total des monomères.

Selon un mode particulièrement préféré du procédé conforme à l'invention, la polymérisation est conduite à 78°C, sous un reflux de l'éthanol, dont la concentration est comprise entre 30 et 60 %, et préférentiellement voisine de 50%, en suivant le protocole suivant :

- on introduit l'acide acrylique en premier dans un réacteur, le temps d'introduction étant compris entre 20 et 45 minutes et, préférentiellement, voisin de 30 minutes, avec 18 à 20 % de la quantité totale de l'initiateur ;
- on laisse polymériser à 78° C, pendant 15 à 45 minutes et, préférentiellement, pendant 30 minutes ;
- on introduit, ensuite, dans le réacteur, un mélange de monomères durs et mous, le temps d'introduction étant compris entre 1 heure et 2 heures et, préférentiellement, voisin de 1 heure 30, avec 80 à 82 % de la quantité totale de l'initiateur ;
- on laisse polymériser 3 à 4 heures supplémentaires pour assurer la consommation complète des monomères.
- on dilue le milieu réactionnel par de l'éthanol, pour obtenir un polymère filmogène à 20 % de concentration.

Un autre objet de l'invention concerne des polymères filmogènes susceptibles d'être obtenus conformément au procédé conforme à l'invention de préparation de polymères filmogènes.

Dans les compositions conformes à l'invention, le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en $C_1$-$C_4$.

Parmi ces alcools, on peut citer l'éthanol, l'isopropanol. L'éthanol est particulièrement préféré.

La composition de l'invention peut également contenir au moins un additif choisi parmi les épaississants, les tensioactifs anioniques, non ioniques, cationiques ou amphotères, les parfums, les conservateurs, les filtres solaires, les protéines, les vitamines, les provitamines, les polymères fixants ou non fixants anioniques, non ioniques, cationiques ou amphotères, les huiles minérales, végétales ou synthétiques, les céramides, les pseudocéramides, les silicones volatiles ou non, linéaires ou cycliques, modifiées ou non et tout autre additif classiquement utilisé dans les compositions cosmétiques destinées, notamment, à être appliquées sur les cheveux.

De préférence, on utilisera, comme additif, un polymère fixant anionique, siliconé ou non.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas ou substantiellement pas, altérées par l'addition envisagée.

Ces compositions peuvent se présenter sous diverses formes, comme des lotions, des gels, des laits, des crèmes, des mousses et peuvent, en particulier être appliquées à partir de flacons pompes ou dans des récipients aérosols, afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux. Les compositions conformes à l'invention peuvent aussi se présenter sous la forme de crèmes, de gels, d'émulsions, de lotions ou de cires.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyl éther.

Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.

Les compositions de l'invention peuvent être utilisées dans des produits capillaires, des rouges à lèvres, des vernis à ongles ou des crèmes de soin.

De préférence, les compositions conformes à l'invention sont appliquées sur des cheveux, qu'ils soient secs ou humides.

L'invention va être plus complètement illustrée à l'aide des exemples non limitatifs suivants.

Tous les pourcentages sont des pourcentages relatifs en poids par rapport au poids total de la composition et m.a. signifie matière active.

EXEMPLE:

On réalise trois compositions conformes à l'invention.

I/ Préparation des polymères filmogènes

1/ Préparation du polymère filmogène P1

Dans un réacteur de 1 litre muni d'une agitation centrale, d'un réfrigérant et d'une arrivée d'azote, on introduit 140g d'éthanol absolu que l'on porte à reflux (78° C) en 45 minutes. A reflux, on introduit dans le réacteur, simultanément et, en 2 heures, les coulées suivantes :

- 60g d'éthanol absolu et 2.2g de 2,5-bis(2-éthylhexanolperoxy)-2,5-diméthylhexane
- 32g d'acide acrylique + 68g de méthacrylate de méthyle + 100g d'acrylate d'isobutyle.

A la fin des 2 ajouts, on laisse 3 heures supplémentaires à 78°C puis on laisse revenir sous agitation à la température ambiante. On obtient une solution visqueuse de polymère P1 à 50 % de concentration dans l'éthanol absolu. La composition du polymère P1 est, en pourcentage en poids par rapport au poids total de polymère :

| | |
|---|---|
| Acide acrylique | 16 % |
| Méthacrylate de méthyle | 34 % |
| Acrylate d'isobutyle | 50 % |

2/ Préparation du polymère filmogène P2

On utilise une voie de synthèse identique à la précédente, sauf la coulée des monomères, qui est la suivante: 32g d'acide acrylique + 70g de méthacrylate de méthyle + 29g d'acrylate de méthyle + 20g d'acrylate d'isobutyle. On obtient une solution visqueuse de polymère P2 à 50 % de concentration dans l'éthanol absolu. La composition du polymère P2 est, en pourcentage en poids par rapport au poids total de polymère :

| | |
|---|---|
| Acide acrylique | 16 % |
| Méthacrylate de méthyle | 35 % |
| Acrylate d'isobutyle | 20 % |
| Acrylate de méthyle | 29 % |

3/ Préparation du polymère filmogène P3

Le polymère P3 est réalisé en mettant en oeuvre le procédé conforme à l'invention de fabrication de polymère filmogène. Dans un réacteur de 1 litre muni d'une agitation centrale, d'un réfrigérant et d'une arrivée d'azote, on introduit 140g d'éthanol absolu que l'on porte à reflux (78° C) en 45 minutes. On introduit au reflux et en 45 minutes le mélange suivant : 32g d'acide acrylique + 0.4g d'initiateur + 10g d'éthanol. On laisse 15 minutes au reflux après la fin de la coulée. On introduit ensuite toujours au reflux et en 1h30 le mélange suivant : 70g de méthacrylate de méthyle + 58g d'acrylate de méthyle + 40g d'acrylate d'isobutyle + 50g de 510A + 1.8 g de d'initiateur. On laisse 3 heures au reflux après la fin de la coulée et on dilue le milieu réactionnel pour avoir une concentration finale voisine de 20 % dans l'éthanol. On obtient ainsi le polymère P3. La composition du polymère P3 est, en pourcentage en poids par rapport au poids total de polymère :

| | |
|---|---|
| Acide acrylique | 16 % |
| Méthacrylate de méthyle | 35 % |
| Acrylate d'isobutyle | 20 % |
| Acrylate de méthyle | 29 % |

II/ Préparation des compositions

| Composition 1 : | |
|---|---|
| Polymère P1 2-amino-2-méthyl-1-propanol Ethanol | 6 % m.a. qs 100% neutralisation qsp 100 g |

| Composition 2 : | |
|---|---|
| Polymère P2 2-amino-2-méthyl-1-propanol Ethanol | 6 % m.a. qs 100% neutralisation qsp 100 g |

| Composition 3 : | |
|---|---|
| Polymère P3 2-amino-2-méthyl-1-propanol Ethanol | 6 % m.a. qs 100% neutralisation qsp 100 g |

III/ Caractérisation des compositions

Les paramètres définissant le profil mécanique des compositions sont rassemblés dans le tableau I ci-après. L'indice acide figure également dans ce tableau I.

Tableau I

| Composition | Indice acide | Allongement $\varepsilon$ (en %) | Recouvrance instantannée Ri (en %) | Recouvrance à 300 secondes, $R_{300}$ (en %) |
|---|---|---|---|---|
| 1 | 124 | >1500 | 25 | 35 |
| 2 | 124 | 1200 | 35 | 55 |
| 3 | 124 | 1200 | 48 | 61 |

On applique les compositions 1 à 3 conformes à l'invention sur des mèches de cheveux naturels eurochâtains.
Après conditionnement de ces compositions dans des flacons pompes et applications de 10 pressions sur mèches de cheveux naturels de 5 grammes, préalablement lavés et séchés, on constate que ces compositions fixent bien la coiffure, donnent aux cheveux un toucher gainé agréable et s'éliminent bien au shampooing.

**Revendications**

1. Composition cosmétique comprenant, dans un milieu cosmétiquement acceptable, au moins un polymère filmogène (A) choisi de telle sorte que le matériau obtenu par séchage de ce polymère en milieu aqueux ou alcoolique, à température ambiante et à un taux d'humidité relative de 50 %, présente un profil mécanique défini par au moins:

   (a) un taux d'allongement à la rupture ($\varepsilon_r$) supérieur ou égal à 160 %;
   (b) une recouvrance instantanée ($R_i$) comprise entre 25 et 70 % après un allongement de 150 %,
   (c) une recouvrance ($R_{300}$) à 300 secondes comprise entre 30 et 100 % après un allongement de 150 % ;

   le polymère filmogène (A) contenant au moins un monomère à motif acide insaturé, dont l'indice acide est supérieur à 82.

2. Composition selon la revendication 1, **caractérisée par le fait que** l'indice acide est compris entre 82 et 235, et plus préférentiellement entre 90 et 190, et encore plus préférentiellement entre 100 et 130.

3. Composition selon l'une quelconque des revendications précédentes, **caractérisée par le fait que** ou les polymères filmogènes (A) sont, de préférence, présents à des concentrations comprises entre 0,05 et 20 % en poids, plus préférentiellement comprises entre 0,1 et 15 % en poids, et plus préférentiellement entre 0,25 et 10 % en poids par rapport au poids total de la composition.

4. Composition selon l'une quelconque des revendications précédentes, **caractérisée par le fait que** le polymère filmogène (A) est choisi parmi ceux possédant, parmi leurs monomères, au moins un acide insaturé carboxylique ou sulfonique.

5. Composition selon l'une quelconque des revendications précédentes, **caractérisée par le fait qu'**elle contient en outre des additifs cosmétiques conventionnels choisis parmi les épaississants, les tensioactifs anioniques, non ioniques, cationiques ou amphotères, les parfums, les conservateurs, les filtres solaires, les protéines, les vitamines, les provitamines, les polymères fixants ou non fixants anioniques, non ioniques, cationiques ou amphotères, les huiles minérales, végétales ou synthétiques, les céramides, les pseudocéramides, les silicones volatiles ou non, linéaires ou cycliques, modifiées ou non et tout autre additif classiquement utilisé dans les compositions cosmétiques destinées à être appliquées sur les cheveux.

6. Dispositif aérosol contenant une composition selon l'une quelconque des revendications précédentes.

7. Procédé de maintien ou de mise en forme de la coiffure, **caractérisé par le fait qu'**il comprend la mise en oeuvre d'une composition conforme à l'une quelconque des revendications 1 à 5.

8. Procédé de fabrication de polymères filmogènes, **caractérisé par le fait qu'**on effectue les opérations suivantes, dans un milieu solvant alcoolique ou hydroalcoolique, en présence d'un initiateur de radicaux libres, à une température comprise entre 60 °C et 90 °C :

   - on polymérise, d'abord, des monomères (i) à motif(s) hydrophile(s), afin d'obtenir un polymère riche en motif(s) hydrophile(s) ;
   - on polymérise, ensuite, le polymère riche en motif(s) hydrophile(s) ainsi obtenus avec un mélange de monomères durs (ii) et de monomères mous (iii), afin d'obtenir le polymère filmogène ;

   les monomères (i), (ii) et (iii) étant mis en oeuvre dans les proportions suivantes, définies en poids par rapport au poids total des monomères dans le polymère filmogène :

   - 10 à 50 % de monomères (i) à motif(s) hydrophile(s) ;
   - 20 à 50 % de monomères durs (ii), et
   - 30 à 70 % de monomères mous (iii).

9. Procédé selon la revendication 8, **caractérisé par le fait que** les monomères (i) à motif(s) hydrophile(s) sont choisis parmi l'acide acrylique, l'acide méthacrylique, l'acide itaconique, et préférentiellement l'acide acrylique.

10. Procédé selon l'une quelconque des revendications 8 ou 9, **caractérisé par le fait que** le monomère (ii) dur est le méthacrylate de méthyle.

11. Procédé selon l'une quelconque des revendications 8 à 10, **caractérisé par le fait que** le monomères mous (iii) est choisi parmi les acrylates de méthyle et d'isobutyle.

12. Polymère filmogène susceptible d'être obtenu conformément au procédé selon l'une quelconque des revendications 8 à 11.

13. Composition cosmétique comprenant un polymère conforme à la revendication 12.

14. Utilisation d'une composition selon l'une quelconque des revendications 1 à 5 ou 13 en cosmétique, notamment dans des produits capillaires, des rouges à lèvres, des vernis à ongles ou des crèmes de soin.

15. Utilisation d'une composition selon l'une quelconque des revendications 1 à 5 et 13, pour la fabrication d'un produit cosmétique, notamment des compositions capillaires destinées au maintien ou à une mise en forme de la coiffure.

**Office européen des brevets**

**RAPPORT PARTIEL DE RECHERCHE EUROPEENNE**

qui selon la règle 45 de la Convention sur le brevet européen est considéré, aux fins de la procédure ultérieure, comme le rapport de la recherche européenne

Numéro de la demande

EP 01 40 2660

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | WO 98 00096 A (OREAL )) 8 janvier 1998 (1998-01-08) | 1-5,7-9, 12-15 | A61K7/06 A61K7/48 |
| A | * revendications; exemples * | 10,11 | |
| | --- | | |
| X | FR 2 697 160 A (OREAL) 29 avril 1994 (1994-04-29) * page 2, ligne 38 - ligne 39 * * page 3, ligne 1 - ligne 4 * * revendications 1-3,5,14 * | 1-5,7, 14,15 | |
| | --- | | |
| X | US 4 117 853 A (REESE GUNTER ET AL) 3 octobre 1978 (1978-10-03) * colonne 8, ligne 25 - ligne 25 * * revendications * | 1-5,7, 14,15 | |
| | --- | | |
| A | WO 00 30594 A (OREAL) 2 juin 2000 (2000-06-02) * le document en entier * | 1-7,14, 15 | |
| | --- | | |
| A | WO 00 30593 A (OREAL) 2 juin 2000 (2000-06-02) * le document en entier * --- -/-- | 1-7,14, 15 | **DOMAINES TECHNIQUES RECHERCHES (Int.Cl.7)** A61K C08F |

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| BERLIN | 18 janvier 2002 | Angiolini, D |

**Office européen des brevets**

**RECHERCHE INCOMPLETE
FEUILLE SUPPLEMENTAIRE C**

Numéro de la demande

EP 01 40 2660

Revendications ayant fait
l'objet de recherches complètes:
    8-13

Revendications ayant fait
l'objet de recherches incomplètes:
    1-7 14 15

Raison pour la limitation de la recherche:

Les revendications 1-7,14,15 présentes ont trait à une composition
définie en ce qu'elle comprend un polymère (A) défini au moyen des
paramètres suivants:

le profil mécanique du film obtenu par séchage de ce polymère en milieu
aqueux ou alcoolique, à température ambiante et à un taux d'humidité
relative de 50%, défini par au moins:
a) un taux d'allongement à la rupture supérieur ou égal a 160%;
b) unr recouvrance instantanée comprise entre 25 et 70% après un
allongement de 150%,
c) une recouvrance à 300 secondes comprise entre 30 et 100% après un
allongement de 150%.

Il est impossible de comparer le polymère défini par les paramètres
choisis par le déposant, avec ceux révélés sans paramètres dans l'état de
la technique. L'utilisation de ces paramètres est considérée, dans le
présent contexte, comme ménant à un manque de clarté au sense de
l'Article L.616-6 CPI.
Le manque de clarté qui en découle est tel qu'une recherche significative
complète est impossible.
Par conséquent, la recherche a été limitée aux documents citant les
paramètres physiques mentionnés par le déposant et en outre elle a été
basée sur l'idée inventive générale, en particulier sur les examples.

**Office européen**
**des brevets**

**RAPPORT PARTIEL**
**DE RECHERCHE EUROPEENNE**

Numero de la demande

EP 01 40 2660

| | DOCUMENTS CONSIDERES COMME PERTINENTS | | CLASSEMENT DE LA DEMANDE (Int.Cl.7) |
|---|---|---|---|
| Catégorie | Citation du document avec indication, en cas de besoin, des parties pertinentes | Revendication concernée | |
| A | FR 2 786 391 A (OREAL) 2 juin 2000 (2000-06-02) * revendications 1-3,8-11 * --- | 1,4-15 | |
| A | FR 2 786 392 A (OREAL) 2 juin 2000 (2000-06-02) * page 7, ligne 20 - ligne 25; revendications 1,3,4,7-10 * ----- | 1,3,5-8, 10-15 | |
| | | | **DOMAINES TECHNIQUES RECHERCHES** (Int.Cl.7) |

EPO FORM 1503 03.82 (P04C11)

**ANNEXE AU RAPPORT DE RECHERCHE EUROPEENNE
RELATIF A LA DEMANDE DE BREVET EUROPEEN NO.**             EP 01 40 2660

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-01-2002

| Document brevet cité au rapport de recherche | | | Date de publication | Membre(s) de la famille de brevet(s) | | Date de publication |
|---|---|---|---|---|---|---|
| WO 9800096 | A | | 08-01-1998 | FR | 2750600 A1 | 09-01-1998 |
| | | | | EP | 0847270 A1 | 17-06-1998 |
| | | | | WO | 9800096 A1 | 08-01-1998 |
| | | | | JP | 10511406 T | 04-11-1998 |
| | | | | US | 5965116 A | 12-10-1999 |
| FR 2697160 | A | | 29-04-1994 | FR | 2697160 A1 | 29-04-1994 |
| | | | | AT | 152344 T | 15-05-1997 |
| | | | | AU | 667246 B2 | 14-03-1996 |
| | | | | AU | 5424094 A | 24-05-1994 |
| | | | | BR | 9305699 A | 31-12-1996 |
| | | | | CA | 2117355 A1 | 11-05-1994 |
| | | | | DE | 69310341 D1 | 05-06-1997 |
| | | | | DE | 69310341 T2 | 20-11-1997 |
| | | | | EP | 0618793 A1 | 12-10-1994 |
| | | | | ES | 2101357 T3 | 01-07-1997 |
| | | | | WO | 9409749 A1 | 11-05-1994 |
| | | | | JP | 2710691 B2 | 10-02-1998 |
| | | | | JP | 7502761 T | 23-03-1995 |
| | | | | NZ | 257580 A | 26-07-1995 |
| | | | | US | 5660820 A | 26-08-1997 |
| | | | | US | 5709850 A | 20-01-1998 |
| | | | | ZA | 9308060 A | 07-06-1994 |
| US 4117853 | A | | 03-10-1978 | DE | 2453629 A1 | 13-05-1976 |
| | | | | FR | 2290890 A1 | 11-06-1976 |
| | | | | GB | 1517789 A | 12-07-1978 |
| | | | | IT | 1048655 B | 20-12-1980 |
| | | | | JP | 51070830 A | 18-06-1976 |
| | | | | NL | 7512108 A | 14-05-1976 |
| WO 0030594 | A | | 02-06-2000 | FR | 2786391 A1 | 02-06-2000 |
| | | | | AU | 1276700 A | 13-06-2000 |
| | | | | BR | 9907723 A | 17-10-2000 |
| | | | | CN | 1295459 T | 16-05-2001 |
| | | | | EP | 1051146 A1 | 15-11-2000 |
| | | | | WO | 0030594 A1 | 02-06-2000 |
| | | | | PL | 341980 A1 | 07-05-2001 |
| WO 0030593 | A | | 02-06-2000 | FR | 2786392 A1 | 02-06-2000 |
| | | | | AU | 1276600 A | 13-06-2000 |
| | | | | BR | 9907724 A | 17-10-2000 |
| | | | | CN | 1295458 T | 16-05-2001 |
| | | | | EP | 1051145 A1 | 15-11-2000 |
| | | | | WO | 0030593 A1 | 02-06-2000 |
| | | | | PL | 341932 A1 | 07-05-2001 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE
RELATIF A LA DEMANDE DE BREVET EUROPEEN NO.**     EP 01 40 2660

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-01-2002

| Document brevet cité au rapport de recherche | | Date de publication | Membre(s) de la famille de brevet(s) | | Date de publication |
|---|---|---|---|---|---|
| FR 2786391 | A | 02-06-2000 | FR | 2786391 A1 | 02-06-2000 |
| | | | AU | 1276700 A | 13-06-2000 |
| | | | BR | 9907723 A | 17-10-2000 |
| | | | CN | 1295459 T | 16-05-2001 |
| | | | EP | 1051146 A1 | 15-11-2000 |
| | | | WO | 0030594 A1 | 02-06-2000 |
| | | | PL | 341980 A1 | 07-05-2001 |
| FR 2786392 | A | 02-06-2000 | FR | 2786392 A1 | 02-06-2000 |
| | | | AU | 1276600 A | 13-06-2000 |
| | | | BR | 9907724 A | 17-10-2000 |
| | | | CN | 1295458 T | 16-05-2001 |
| | | | EP | 1051145 A1 | 15-11-2000 |
| | | | WO | 0030593 A1 | 02-06-2000 |
| | | | PL | 341932 A1 | 07-05-2001 |

EPO FORM P0460

Pour tout renseignement concernant cette annexe : voir Journal Officiel de l'Office européen des brevets, No.12/82